Europäisches Patentamt

European Patent Office

Office européen des brevets

(11) Veröffentlichungsnummer: **0 501 376 A2**

## (12) EUROPÄISCHE PATENTANMELDUNG

(21) Anmeldenummer: **92103063.1**

(22) Anmeldetag: **24.02.92**

(51) Int. Cl.⁵: **C12M 1/00**

(30) Priorität: **25.02.91 DE 4105841**
**31.12.91 DE 4143229**

(43) Veröffentlichungstag der Anmeldung:
**02.09.92 Patentblatt 92/36**

(84) Benannte Vertragsstaaten:
**AT BE CH DE DK ES FR GB GR IT LI LU MC
NL PT SE**

(71) Anmelder: **Bauer, Heinrich**

Kurfürstinstrasse 25
**W-8858 Marienheim(DE)**

(72) Erfinder: **Bauer, Heinrich**
**Kurfürstinstrasse 25**
**W-8858 Marienheim(DE)**

(74) Vertreter: **Glöser, Otto**
**Sudetenstrasse 6**
**W-8076 Baar-Ebenhausen 1(DE)**

(54) **Anlage zur Gewinnung von Biogas aus Gülle.**

㊼ Die Erfindung bezieht sich auf eine Anlage zur Gewinnung von Biogas aus Gülle, die für den Gärprozeß einen beheizten Fermenter (1) aufweist, der in seiner Wirkung dadurch verbessert werden soll, daß er mit mindestens einer den Güllefluß in Gegenrichtung umlenkenden, auf dem Fermenterboden (vgl. 19) senkrecht stehenden Trennwand (2) ausgestattet ist, die von der Innenseite (vgl. 3) der einen Fermenterstirnwand (4) ausgeht, die Innenseite der gegenüberliegenden Fermenterstirnwand (5) jedoch nicht erreicht und die bodennahe Zufuhr (9) für die unvergorene Gülle in den einen, von der Trennwand (2) geschaffenen Teilraum des Fermenters (1) mündet, während der andere Teilraum an die Entnahmestelle (vgl. 28, 29) für die ausgegorene Substanz angeschlossen ist, wobei Zu- und Ablauf (9, 28) in Bodennähe liegen und daß ferner dem Fermenter (1) ein Biogaskühler (41) besonderer Art nachgeschaltet ist, auf den Schadstoffmengen- und/oder Qualitätsmeßgeräte (52), Steuereinrichtungen sowie sonstige den Betrieb der Anlage optimierende Einrichtungen folgen.

Fig.2

Die Erfindung bezieht sich auf eine Anlage zur Gewinnung von Biogas aus Gülle, die für den Gärprozeß einen beheizten Behälter, einen sogenannten Fermenter aufweist, der die Gülle unter Einhaltung einer bestimmten Verweilzeit aufnimmt und an einen Biogasbehälter und an eine Entnahmestelle für die ausgegorene Substanz angeschlossen ist.

Anlagen der erwähnten Art sind seit längerer Zeit bekannt, so z.B. aus den DE-OS 29 44 584 und der DD-PS 226 550. Die Wirtschaftlichkeit solcher Anlagen läßt aber zu wünschen übrig. In diesem Zusammenhang wird auf einen die Unwirtschaftlichkeit bestätigenden Artikel auf den Seiten 24 und 25 des "Landwirtschaftlichen Wochenblattes", Heft 22 vom 30.05.1987 verwiesen. Weitere Auskünfte über den unbefriedigenden Stand der Technik geben die Seiten 32 und 33 des gleichen "Wochenblattes" im Heft 27 vom 09.07.1988. Eine positive Beurteilung einer Biogasanlage ist auf seite 37 des "Landwirtschaftlichen Wochenblattes", Heft 6 vom 10.02.1990 nachlesbar.

In der DE-OS 37 15 952.6 ist ein Festbett-Fermenter beschrieben, der hier aber insofern von untergeordneter Bedeutung ist, da bei der eingangs angesprochenen Anlage der erwähnte Behälter keinen Festbett-, sondern einen Durchlauf-Fermenter darstellt.

Wenn man den bekannten Stand der Technik würdigt, ist festzustellen, daß die Unwirtschaftlichkeit nicht nur auf einen zu hohen Verschleiß der Anlagenteile, sondern auch auf einen zu hohen Eigenbedarf an Energie und vor allem auf eine ungenügende Gasausbeute zurückzuführen ist, die ihrerseits auf einem unvollkommenen Gärprozeß beruht. Ein weiterer Nachteil der bekannten Anlagen ist der Fermenter selbst, der, insbesondere in kleineren Betrieben, zuviel Raum verbraucht, der meistens nur bedingt, wenn überhaupt zur Verfügung steht. Ein freistehender Fermenter ist auch mitverantwortlich dafür, daß - wie schon angedeutet - zuviel Eigenbedarf an Wärmeenergie anfällt.

Demgegenüber verfolgt die vorliegende Erfindung das Ziel, eine Anlage zu schaffen, deren Fermenter einen vollkommenen Gärprozeß sicherstellt, einen geringen Eigenbedarf an Energie hat und so untergebracht ist, daß er nicht stört. Darüber hinaus soll die Anlage, nicht nur wirtschaftlich arbeiten, sondern auch wirtschaftlich, also mit einem Verhältnismäßig geringen Aufwand herstellbar, nicht störanfällig und als Ganzes langlebig sein.

Dieses Ziel und insbesondere die Schaffung eines den Anforderungen entsprechenden Fermenters läßt sich dann erreichen, wenn man sich die Lehre des kennzeichnenden Teiles des Hauptanspruches zunutze macht. Der bevorzugten einzigen Trennwand kommt insofern eine besondere Bedeutung zu, als sie trotz einer verhältnismäßig kleinen Länge des rechteckigen Fermenters einen langen Gärweg schafft, der mit dazu beiträgt, daß bei einer ausreichenden Verweilzeit eine vollkommene Vergärung der zugeführten Gülle eintritt. Die Trennwand im Zusammenwirken mit der besonderen Anordnung von Zu- und Ablauf bewirkt, daß eine vermengung von Frischgülle mit der vergorenen Substanz unmöglich ist, d.h., es kann nicht vorkommen, daß, wie bei bekannten Fermentern, Frischgülle mehr oder weniger unvergoren in den Ablauf gelangt und dort zur Bildung von Schlamm führt, der erst aufgerührt werden muß, wenn seine Entfernung ansteht. Bei dem erfindungsgemäßen Fermenter ist die Vergärung so gut, daß Schlamm nicht mehr anfällt und die ausgegorene Gülle eine homogene, nahezu geruchslose Flüssigkeit ist, die mühelos abgepumpt und sofort aufs Feld gebracht oder zwischengelagert werden kann, was von den für das Wachstum wichtigen Zeiträumen bestimmt wird. Die Geruchslosigkeit beruht darauf, daß der in der Rohgülle enthaltene und besonders geruchsintensive Ammoniak in Ammonium umgewandelt wird und der Gärprozeß auch andere übelriechende Stoffe geruchsmäßig gewissermaßen entspannt. Die sofortige Verwendbarkeit wiederum ist darauf zurückzuführen, daß die Aggressivität der Gülle abgebaut ist, also eine Art ungefährlicher Kopfdünger gewonnen wurde. Diese vorteilhaften Eigenschaften der aus dem erfindungsgemäßen Fermenter austretenden Gülle sind, um die Wichtigkeit nochmals zu betonen, nur dann zu erreichen, wenn im Fermenter eine vollkommene Vergärung stattgefunden hat. Selbstverständlich ist es dazu auch erforderlich, daß bestimmte Verweilzeiten eingehalten werden und der Gärprozeß unter bestimmten Temperaturen stattfindet. Die Tatsache, daß bei dem erfindungsgemäßen Fermenter mindestens drei unterschiedlich temperierbare Heizzonen vorhan-den sind, kann z.B. bei einer kontinuierlichen temperatursteigerung in der Vor- und Hauptgärzone erreicht werden, daß es zu einer Beschleunigung des Gärprozesses kommt, wodurch sich die Verweilzeiten abkürzen lassen und so eine Steigerung des Leistung des fermenters eintritt. Da die Heizzonen - wie schon angedeutet - unabhängig voneinander mit Wärmeenergie versorgt werden, ist es dennoch möglich, den Gärprozeß zu beeinflussen und/oder die Anlage den Witterungsverhältnissen anzupassen, wobei es denkbar ist, daß in der Nachgärzone die niedrigste Temperatur herrscht. Eine pneumatische Umwälzung schließlich hat den Vorteil, daß sie einmal ohne bewegliche Teile arbeitet und so gut wie ohne Verschleiß bleibt und auch keiner Korrosion unterliegt, und zum anderen die sich im Gärprozeß befindliche Gülle durch und durch erwärmt wird, was wiederum den Gärvorgang als solchen begünstigt.

Wirtschaftlich von Vorteil ist es ferner, wenn die Maßnahme gemäß Anspruch 2 zur Anwendung kommt. Ähnliches gilt für die Lehre nach Anspruch 3, die auch noch den Gärprozeß begünstigt, indem eine Art Impfung stattfindet.

Wenn man nach den Maßnahmen gemäß Anspruch 4 vorgeht, erhält man einen kleinbauenden Fermenter, in welchem ein Biogasspeicher integriert ist. Durch die Abdeckelung des Fermenters ergibt sich eine Bauart, die geschlossen ist und die Einbauten mit Sicherheit vor Beschädigungen schützt, andererseits aber das Innere des Fermenters zugänglich macht, wenn dies einmal erforderlich sein sollte.

Wenn beabsichtigt ist, z.B. wegen Veränderung des Viehbestandes die Wirkung der Trennwand zu verändern, wird auf den Anspruch 5 aufmerksam gemacht.

Um einmal vernünftige Gärwege zu erhalten und zum anderen Korrosionsgefahren und Wärmeverluste unmöglich zu machen, wird auf die Maßnahme nach Anspruch 6 verwiesen.

Im Hinblick auf geringe Wärmeverluste und aus Gründen der nicht störenden Unterbringung des Fermenters wird die Lösung nach Anspruch 7 vorgeschlagen.

Für das Abpumpen der ausgegorenen Gülle ist es zweckmäßig, sich die Maßnahmen nach Anspruch 8 zunutze zu machen. Dabei ist es auch von Vorteil, daß man durch den Einstieg in das von der Folienhaube abgedeckte Innere des Fermenters gelangen kann.

Für die Weiterverarbeitung bzw. für die problemlose Nutzung des Biogases ist es zweckmäßig, nach Anspruch 9 vorzugehen, zumal es dadurch möglich ist, die Umwelt belastende und/oder korrosionsfördernde Stoffe, z.B. Schwefel, durch Kondensation auszuscheiden.

Eine Vervollkommnung der Umweltfreundlichkeit, der Langlebigkeit und der Korrosionsfestigkeit ist dann zu erreichen, wenn man nach der Lehre des Anspruches 10 handelt. Durch diese Maßnahmen gelingt es nämlich, durch Kondensation den Schwefel und auch andere Schadstoffe aus dem Biogas soweit auszuscheiden, daß Korrosionsgefahren für einen mit Biogas zu betreibenden Gasmotor vermieden sind und daher Laufleistungen erzielt werden können, welche die Laufleistungen von Kraftfahrzeugmotoren übersteigt. Der ausgeschiedene Schwefel kann auch der Umwelt nicht gefährlich werden, da er im Kondensat aufgrund der stetig ansteigenden Biogasführung, also durch das Vorhandensein eines Gefälles dem Fermenter wieder zufließt und nicht umweltbelastend in Erscheinung tritt, vielmehr als chemisch umgewandelte Substanz in der ausgegorenen und aufs Feld gebrachten Gülle wachstumsfördernd wirkt.

Eine hohe Leistung des Biogaskühlers ist dann zu erreichen, wenn man nach den Lehren der Ansprüche 11 und 12 vorgeht. Ob man eine oder zwei Rohrwendel vorsieht, ist in der Hauptsache eine Frage der Konstruktion und des zur Verfügung stehenden Raumes. Daß die Rohrwendel aus korrosionsfestem Material besteht, zumindest aber korrosionsfest ausgekleidet ist, versteht sich nahezu von selbst. Als ideales Material kann man, wenn man die Kosten nicht scheut, Edelstahlführungen beliebigen Querschnitts ansehen. Einen geringeren Aufwand erfordern feuerverzinkte Konstruktionen. Ob man eine oder zwei Rohrwendel vorsieht oder z.B. Viereckquerschnitte wählt, ist wiederum eine Konstruktions- und/oder Raumfrage. Als bevorzugt können übliche Rohrquerschnitte angesehen werden. Ähnliches gilt für die entweder axial verlaufenden oder die Biogasführung schlangenlinienförmig umgebenden Rippen o. dgl.. Wichtig ist, daß ein rascher Wärmeaustausch stattfindet, der mit einer Trocknung des Gases parallel läuft.

Aus wirtschaftlichen Gründen, d.h. um den Eigenbedarf an Energie gering zu halten, ist es zweckmäßig, die Bauart nach Anspruch 13 zu wählen.

Wenn man die Verweilzeit des die Biogasführung durchströmenden Biogases, zumindest in der Anfangsphase der Kühlung erhöhen will, erscheint es zweckmäßig, nach Anspruch 14 vorzugehen. Durch die längere Verweilzeit läßt sich der Kühlvorgang als solcher abkürzen. Gleiches kann man auch erreichen, wenn man die mit dem Kühlmedium in Berührung kommenden Oberflächen der Biogasführung unterschiedlich gestaltet, also z.B. den Durchflußquerschnitt von unten nach oben verringert. Das stetig vorhandene Gefälle wiederum verhindert, daß sich in der Biogasführung Rückstände ansammeln bzw. konzentrieren.

Damit Schäden an den dem Biogaskühler nachgeschalteten Aggregaten ausgeschlossen sind und auch die Qualität des Gases kontrollierbar ist, empfiehlt sich die Maßnahme nach Anspruch 15.

Da es stets von Vorteil ist, wenn die Biogaserzeugung zumindest für eine zusammenhängende Zeitspanne kontinuierlich abläuft und auch eine ausreichende Verweilzeit der Gülle in dem Fermenter gesichert erscheint, wird die Maßnahme nach Anspruch 16 vorgeschlagen. Mit der Kontinuität in Verbindung mit der ausreichenden Verweilzeit werden die durch die Maßnahmen des Hauptpatents erzielten Vorteile noch weiter vertieft.

Ein Höchstmaß an Leistung des Fermenters, d.h. eine vollkommene Vergärung der dem Fermenter zugeführten Gülle ist dann zu erreichen, wenn man sich die Lehre des Anspruchs 17 zunutze macht. Für die Praxis bedeutet dies, daß die Frischgülle gewissermaßen mit der vergorenen Substanz geimpft wird, wodurch mit Hilfe der in der vergorenen Substanz befindlichen Bakterien der Gärprozeß im Fermenter von Anfang an intensiver abläuft als ohne Impfung. Das Umpumpen der ausgegorenen Substanz in die Frischgülle bereitet dabei keinerlei Schwierigkeiten, da die vergorene Substanz dünnflüssiger als die unvergorene Gülle ist. Die Menge der umzupumpenden Substanz wird von der Art der Frischgülle bestimmt. Gemäß chemischer Beurteilung müßte die Impfmenge in einem Bereich von 1/2 bis 1 1/2 % der Rohgülle liegen. Die gute Wirkung des Impfens beruht darauf, daß die zu Beginn des Gärvorganges vorhandenen und verhältnismäßig "schwachen Bakterienkulturen" gewissermaßen schlagartig aktiviert werden, also der Gärprozeß von Anfang an schneller abläuft.

Für das Ausfällen von Schwefel, insbesondere auf chemischem Wege, wird die Maßnahme gemäß Anspruch 18 in Vorschlag gebracht.

In der Zeichnung ist die Erfindung beispielsweise veranschaulicht; es zeigen:

Fig. 1 den erfindungsgemäßen Fermenter in Seitenansicht und im Schnitt, wobei der Schnitt längs der Linie I-I der Fig. 2 geführt ist;

Fig. 2 den gleichen Fermenter in Oberansicht und längs der Linie II-II der Fig. 1 geschnitten;

Fig. 3 einen Schnitt längs der Linie III-III der Fig. 2;

Fig. 4 ein Schema der Hauptkomponenten einer Biogasanlage;

Fig. 5 einen schematisiert dargestellten Biogaskühler in Oberansicht, teilweise aufgebrochen;

Fig. 6 ein kurzes Stück der im Biogaskühler befindlichen Rohrwendel, teilweise aufgebrochen, mit möglichen Einzelheiten und

Fig. 7 ein gegenüber Fig. 4 durch besondere Maßnahmen erweitertes Schema der Hauptkomponenten einer Biogasanlage.

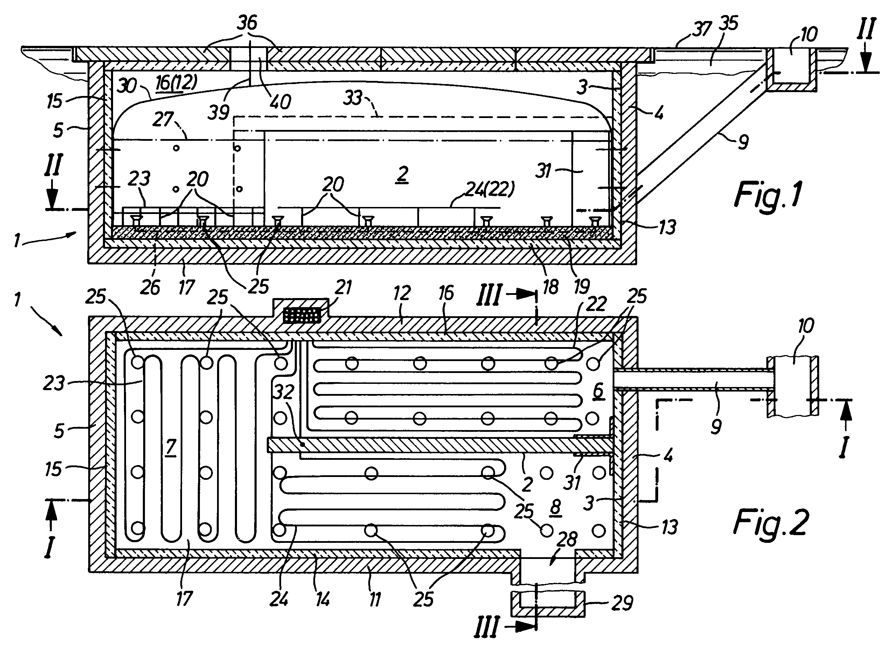

Wie sich aus den stark vereinfacht dargestellten Figuren 1 bis 3 ergibt, hat ein Fermenter 1 in Oberansicht eine rechteckige Form, wobei die Breite in etwa der halben Länge der Rechteckform entspricht. Der Fermenter 1, der aus Beton hergestellt ist, weist eine noch zu konkretisierende Trennwand 2 auf, die mittelbar von der Innenseite 3 der einen Stirnwand 4 des Fermenters 1 ausgeht, die gegenüberliegende Stirnwand 5 jedoch nicht erreicht, so daß sich drei Zonen 6, 7, 8 ergeben, die als Heizzonen genutzt sind, worauf ebenfalls noch näher eingegangen wird.

In die Zone 6, d.h. in den durch die Trennwand 2 gebildeten einen Teilraum mündet ein Güllekanal 9, der von einem Güllesammelkanal 10 eines Stalles für Rinder ausgeht.

Die Stirnwände 4, 5 des Fermenters 1 sowie seine Seitenwände 11, 12 sind mit Isolierplatten 13, 14, 15 und 16 versehen, wie auch der Boden 17 mit einer Isolierplatte 18 ausgestattet ist. Diese Isolierplatte 18 ist mit einem Estrich 19 abgedeckt, der mit nach oben ragenden Stützen 20 ausgestattet ist, auf denen von einem Verteiler 21 aus getrennt voneinander mit Wärmeenergie gespeiste Heizschlangen 22, 23, 24 vorgesehen sind, die es ermöglichen, die Zonen 6, 7 und 8 in einem Bereich von 30 - 39°C unterschiedlich zu temperieren.

Auf dem Estrich 19 sind ferner zahlreiche unter den Heizschlangen 22, 23, 24 liegende Düsen 25 vorgesehen, die an im Estrich 19 vorgesehene Biogas führende Druckleitungen 26 angeschlossen sind und die durch den Zulauf 9 in den Fermenter 1 gelangte Gülle, deren Spiegel mit einer strichpunktierten Linie 27 angegeben ist, durchmischen und so den Gärvorgang intensivieren. Die Düsen 25 haben in den einzelnen Zonen 6, 7, 8, ggf. auch am Ende des Gärweges unterschiedliche Abstände und sie sind außerdem so angeordnet, daß in den einzelnen Temperaturbereichen eine vollkommene und gärungsgerechte Umwälzung stattfindet.

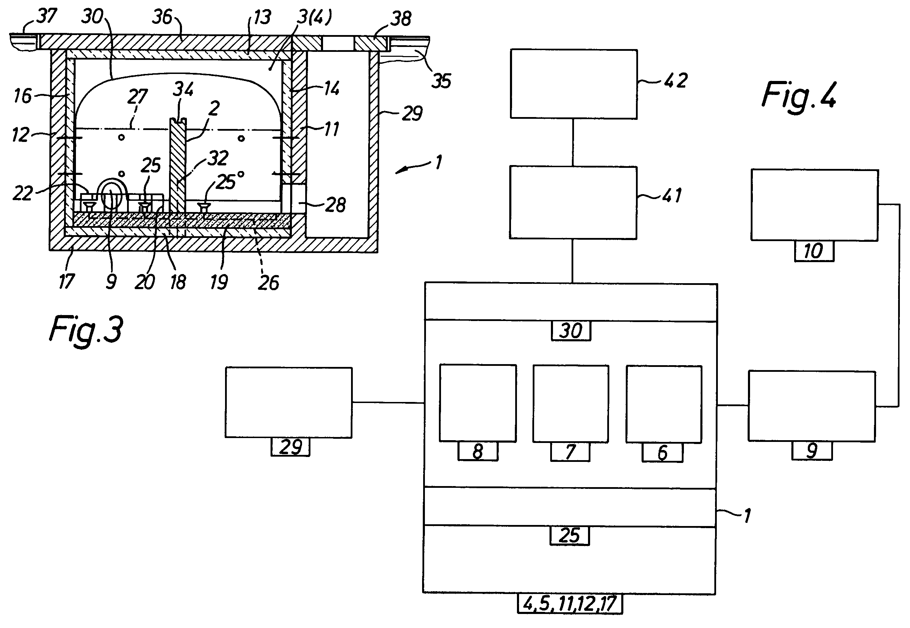

Der Ablauf der vergorenen Gülle erfolgt in Nähe der Fermenterstirnwand 4 bei 28 (vgl. insbesondere Fig. 3), wobei der Ablauf in einen Abpumpbehälter 29 mündet, der ein Teil des Fermenters sein kann, d.h., er kann mit dem Fermenter 1 einstückig aus Beton bestehen. Wichtig ist, daß die Trennwand 2 so in das Fermenterinnere eingesetzt ist, daß die durch den Zulauf 9 in den Fermenter 1 gelangende Gülle nicht unvergoren in den Abpumpbehälter 29 einfließen kann und einen Umweg um die Trennwand 2 machen muß.

Die Trennwand 2 ist von einer verhältnismäßig starken, jedoch flexiblen Folienhaube 30 überspannt, die an den Wänden 4, 5 bzw. 11, 12 des Fermenters 1 unter Zwischenschaltung der Isolierplatten 13 bis 16 befestigt ist, wobei der untere Rand der einen Gasspeicher ergebenden Folienhaube 30 bis weit unter den Güllespiegel 27 reicht (vgl. 22, 23, 24). Die Befestigung erfolgt mit Dübeln, wie dies durch Striche und kleine Kreise in den Figuren 1 und 3 angedeutet ist.

Die Trennwand 2, die 1/3 bis 2/3 der Höhe des Fermenters 1 ausmacht, kann auch in ihrer wirksamen Höhe und Länge veränderbar gestaltet sein, indem sie in eine U-förmige, an der Fermenterstirnwand 4 befestigte und auch die Folienhaube 30 an die Isolierplatte 13 andrückende Halterung 31 eingreift (in Fig. 3 nicht eingezeichnet) und nach links verschoben werden kann. Es versteht sich, daß die Halterung 31 so gestaltet ist, daß ganz gleich, welche Lage die Trennwand 2 einnimmt, keine unvergorene Gülle in den Abpumpschacht 29 gelangen kann. Zur Sicherung der Lage der Trennwand 2 sind Steckverbindungen 32 zum Estrich 19 denkbar, wie eine solche in Figur 2 angedeutet ist.

Wenn man die Höhe der Trennwand 2 verändern will, kann anstatt des in Figur 3 dargestellten Schutzköders für die Folienhaube 30 ein Aufsatzstück aufgesetzt werden, wie dies gestrichelte Linien 33 erkennen lassen. Auch hier können Steckverbindungen zur Anwendung kommen, es sei denn, daß man eine Art Nut-Federverbindung 34 - wie in Fig. 3 gezeigt - vorsieht, die auch für die Bodenhalterung denkbar erscheint. Ebenso könnte die Trennwand 2 (vgl. Fig. 3) einstückig mit dem Boden 17 des Fermenters 1 hergestellt sein, wobei jedoch wegen der Andübelung der Folienhaube 30 die Trennwand 2 von der Isolierplatte 13 bzw. der Fermenterstirnwand 4 einen Abstand haben muß, der zu schließen ist, z.B. mit Dichtstreifen, die wie die Halterung 31 wirken.

Der Fermenter 1 ist, wie dies die Figuren 1 und 3 erkennen lassen, in den Boden 35 eingelassen, und zwar so, daß die Deckel 36, die den Fermenter 1 abdecken, mit der Oberseite 37 des Bodens 35 bündig abschließen. Die Deckel 36 sind so stark bzw. so armiert, daß sie befahrbar sind, also der Fermenter 1 praktisch mitten im Hof eines bäuerlichen Anwesens angeordnet sein kann, ohne daß er stört. Zweckmäßigerweise sind auch die Deckel 36 isoliert, so daß Wärmeverluste so gut wie ausgeschlossen sind. Auch der Abpumpschacht 29 ist mit einem befahrbaren Deckel 38 ausgestattet.

Die Folienhaube 30 ist mit mindestens einem Anschluß 39 versehen, um das Biogas zu den Aggregaten der Anlage abzuführen. Die Leitungen für den Verteiler 21 sind unterirdisch geführt, so daß man von dem erfindungsgemäßen Fermenter 1 überhaupt nichts sieht und dies erst recht, wenn der oder die Anschlüsse 39 bzw. ihre weiterführenden Leitungen nicht durch eine Öffnung 40 des Deckels 36 nach oben austreten, sondern in Kanälen verlegt sind.

In das Schema gemäß Figur 4 sind zum besseren Verständnis der Anlage Bezugszahlen wichtiger Teile des Fermenters 1 gemäß den Figuren 1 bis 3 eingetragen. Zusätzlich sind ein Biogaskühler 41 und eine Kraft-Wärme-Koppelung 42 angedeutet.

Wenn man eine Anlage mit dem erfindungsgemäßen Fermenter 1 betreibt, lassen sich bei der Haltung von 50 bis 55 Großvieheinheiten und bei einem Fermenter-Lagerraum von 120 cbm Gülle und 90 cbm Biogas täglich etwa 80 bis 100 cbm Gas nutzbar erzeugen, das - bezogen auf ein Jahr - ausreicht, den gesamten Energiebedarf der Anlage und des landwirtschaftlichen Anwesens an Strom und Wärme zu decken und darüber hinaus jährlich gut 20.000 kw/h in das öffentliche Netz einzuspeisen. In den Sommermonaten ist ein Überschuß an Wärme zu erwarten, der vorteilhaft für eine Futtertrocknung verwendet werden kann.

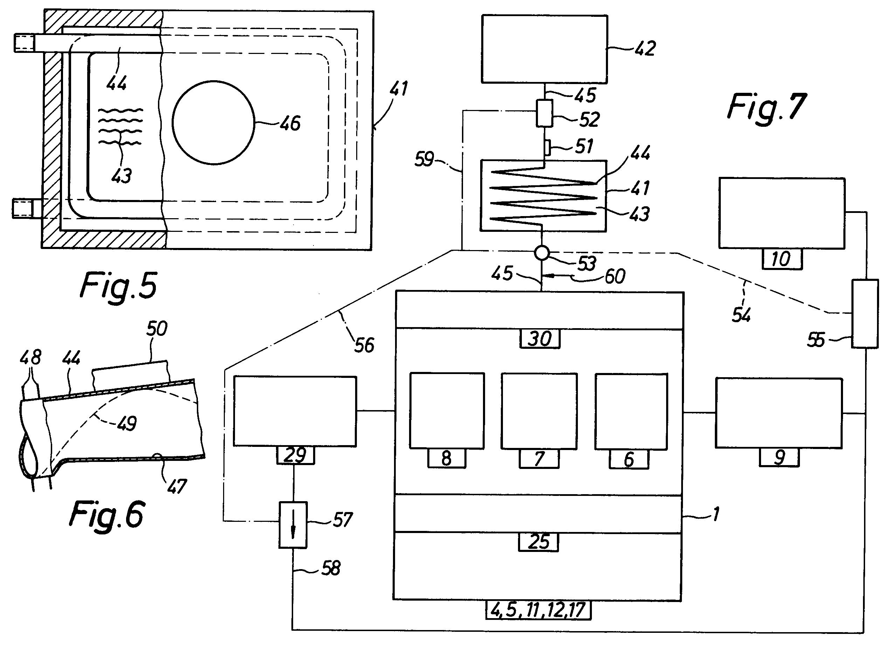

Eine weitere günstige Beeinflussung der erzielten Vorteile ist dann zu erwarten, wenn man die Anlage 1 nach den Figuren 5 bis 7 aufbaut.

In Fig. 5 erkennt man den bereits erwähnten Biogaskühler 41, der stark isoliert ist und Kühlflüssigkeit 43 aufnimmt. In der Kühlflüssigkeit bzw. in dem Biogaskühler 41 befindet sich eine Rohrwendel 44, die ein Zwischenstück der aus Fig. 7 ersichtlichen Biogasleitung 45 zwischen Fermenter 1 und der Kraft-Wärme-Koppelung 42 darstellt. Zentral im Biogaskühler 41 befindet sich das eigentliche Kühlaggregat 46, das nicht näher beschrieben ist, da es Handelsware darstellt. Die Rohrwendel 44 sind so gestaltet, daß stets ein Gefälle für den Abfluß des Kondensats vorhanden ist.

Das aus Fig. 6 ersichtliche und gegenüber Figur 5 stark vergrößerte Stück einer Wendel 44 Läßt eine Bremsstrecke 47 erkennen, welche zwar den Durchfluß des Biogases durch Querschnittsverengung etwas verlangsamt, den restlosen Ablauf des Kondensats aber nicht behindert, wenn auch hier eine Verlangsamung eintritt. Diese Bremsstrecke läßt sich leicht mit einem Preßwerkzeug von außen her erzeugen.

Zum besseren Übergang der Wärme in das Kühlmedium 43 sind ringförmige Rippen 48 vorgesehen, die auch schlangenlinienförmig verlaufen könnten, wie dies die gestrichelte linie 49 andeutet. Ebenso sind axial verlaufende Rippen 50 denkbar. Ebenso ist ein runder Querschnitt nicht zwingend, vielmehr könnten z.B. Vierkantrohre zur Anwendung kommen. Wichtig hingegen ist es, daß zumindest die Innenflächen der Wendel 44 korrosionsfest sind. Ebenso ist Wert darauf zu legen, daß die Biogasleitung 45 bis in den Fermenter 1 ein Gefälle hat, damit das Kondensat mit Sicherheit in den Fermenter 1 zurückfließt.

Der Fig. 7 ist hinter dem Biogaskühler 41 ein Temperaturfühler 51 zu entnehmen, der das Kühlaggregat 46 derart steuert, daß einmal eine günstige Verbrennung in einem Gasmotor stattfindet und zum anderen Energie nicht unnötig verbraucht wird. Daß allerdings die höchstmögliche Wirkung für das Ausscheiden von Schadstoffen gesichert sein muß, versteht sich nahezu von selbst.

In der Biogasleitung 45, zwischen dem Biogaskühler 41 und der Kraft-Wärme-Koppelung 42 ist ein Schadstoffmengen- und/oder Qualitätsmeßgerät 52 vorgesehen, welches dazu beiträgt, daß die ganze Anlage umweltfreundlich und mit einem hohen Wirkungsgrad gefahren werden kann. Letzteres vor allem deshalb, weil sich in der Biogasleitung 45 noch eine Art Gasuhr 53 befindet, die über eine gestrichelt dargestellte Steuerleitung 54 einen gleichmäßigen Zufluß von Frischgülle aus einem Vorratsbehälter 55 in den Güllekanal 9 bzw. in den Fermenter 1 steuert. Außerdem steuert die Gasuhr 53 über eine strichpunktierte Steuerleitung 56 eine Pumpe 57, die in einer die Entnahmestelle 29 mit dem Güllekanal 9 verbindenden Umpumpleitung 58 vorgesehen ist. Die Steuerleitung 56 kann zur Verbesserung des Gärprozesses auch noch mit dem Schadstoffmengen- und/oder Qualitätsmeßgerät 52 über eine Leitung 59 in Verbindung stehen, die mit einer -.-Linie angegeben ist. Mit 60 ist ein Kleinkompressor angedeutet, der dem dosierten Einbringen von Luft in die Biogasleitung 45 dient.

**Patentansprüche**

1. Anlage zur Gewinnung von Biogas aus Gülle, die für den Gärprozeß einen beheizten Behälter, einen sogenannten Fermenter aufweist, der die Gülle unter Einhaltung einer bestimmten Verweilzeit aufnimmt und an einen Biogasbehälter und an eine Entnahmestelle für die ausgegorene Substanz angeschlossen ist, dadurch gekennzeichnet, daß der in Oberansicht eine rechteckige Form aufweisende Fermenter (1) mit mindestens einer den Güllefluß in Gegenrichtung umlenkenden, auf dem Fermenterboden (vgl. 19) senkrecht stehenden Trennwand (2) ausgestattet ist, die von der Innenseite (vgl. 3) der einen Fermenterstirnwand (4) ausgeht, die Innenseite der gegenüberliegenden Fermenterstirnwand (5) jedoch nicht erreicht und die unvergorene Gülle in den einen, von der Trennwand (2) geschaffenen Teilraum des Fermenters (1) mündet (vgl. 9), während der andere Teilraum an die Entnahmestelle (vgl. 28, 29) für die ausgegorene Substanz angeschlossen ist, wobei Zu- und Ablauf (9, 28) in Bodennähe und in oder nahe bei derjenigen Fermenterstirnwand (4) vorgesehen sind, von der die Trennwand (2) ausgeht und daß ferner der umgelenkte Gärweg drei in einer Ebene liegende Heizzonen (6, 7, 8) aufweist, nämlich eine erste Vorgärungszone (vgl. 22) an der Zufuhr (vgl. 9), eine Hauptgärungszone (vgl. 23) in dem von der Tennwand (2) freien Bereich und eine Nachgärungszone (vgl. 24) vor dem Ablauf (28), indem vom Fermenterboden (vgl. 19) beabstandete drei, voneinander unabhängige Heizschlangen (22, 23, 24) o. dgl. vorgesehen sind, die in regelbaren Temperaturbereichen zwischen 30 bis 39° Celsius arbeiten und sich unter den Heizschlangen (22, 23, 24) o. dgl. pneumatische Umwälzdüsen (25) befinden.

2. Anlage nach Anspruch 1, dadurch gekennzeichnet, daß die vom Fermenterboden (vgl. 19) beabstandeten Heizschlangen (22, 23, 24) o. dgl. mit Wärmeenergie aus der Anlage gespeist sind.

3. Anlage nach den Ansprüchen 1 und 2, dadurch gekennzeichnet, daß die unter den Heizschlangen (22, 23, 24) o. dgl. befindlichen pneumatischen Umwälzdüsen (25) an einen mit Biogas betriebenen Kompressor angeschlossen sind.

4. Anlage nach den Ansprüchen 1 bis 3, dadurch gekennzeichnet, daß die Höhe der Trennwand (2) 1/3 bis 2/3 der Höhe des abgedeckelten (vgl. 36) Fermenters (1) ausmacht und in einem Abstand vom Güllespiegel (27) von einer einen Gasspeicher bildenden starken Folienhaube (30) überspannt ist, die mittel- oder unmittelbar an den Innenseiten der Wände (vgl. 4, 5, 11, 12) des Fermenters (1) befestigt ist und mit ihrem unteren Rand bis unter den Güllespiegel (27) reicht und an der höchsten Stelle mindestens einen Anschluß (39) für eine Biogasleitung zu den bekannten Aggregaten der Anlage aufweist.

5. Anlage nach den Ansprüchen 1 bis 4, dadurch gekennzeichnet, daß die Trennwand (2) in ihrer Höhe und/oder in ihrer wirksamen Länge veränderbar gestaltet ist (vgl. dazu 31, 33).

6. Anlage nach den Ansprüchen 1 bis 5, dadurch gekennzeichnet, daß der z.B. doppelt so lang wie breit gestaltete abgedeckelte (vgl. 36) Fermenter (1) aus Beton besteht, dessen Innenseiten mit einer gegen Gülle resistenten Isolierung (13 - 16, 18) versehen sind.

7. Anlage nach den Ansprüchen 1 bis 6, dadurch gekennzeichnet, daß der Fermenter (1) in den Boden (35) eingelassen und überfahrbar gestaltet ist.

8. Anlage nach den Ansprüchen 1 bis 7, dadurch gekennzeichnet, daß der Ablauf (28) für die ausgegorene Gülle in einen Abpumpschacht (29) mündet, dessen Höhe der Fermenterhöhe entspricht, wobei der Ablauf (28) selbst als Einstieg in den Fermenter (1) gestaltet sein kann.

9. Anlage nach den Ansprüchen 1 bis 8, dadurch gekennzeichnet, daß dem von der Folienhaube (30) gebildeten Gasspeicher ein Biogaskühler (41) nachgeschaltet ist.

10. Anlage nach den Ansprüchen 1 bis 9, dadurch gekennzeichnet, daß der Biogaskühler (41) mit einem ein gekühltes (vgl. 46) Kühlmedium (43) enthaltenden und allseits isolierten Behälter ausgestattet ist, der eine von unten nach oben strebende, eine stetige Steigung aufweisende, ein Zwischenstück der Biogasleitung (45) zwischen Fermenter (1) und den Verbrauchern (vgl. 42) darstellende Biogasführung (44) enthält.

11. Anlage nach den Ansprüchen 1 bis 10, dadurch gekennzeichnet, daß die Biogasführung (44) als mindestens eine, in Oberansicht beliebig geführte Rohrwendel beliebigen Querschnitts ausgeführt ist und in den mittigen Innenraum des Behälters ein an sich bekannter Kühleinsatz (46) vorgesehen ist, der bevorzugt die ganze Höhe des Behälters (vgl. 41) einnimmt.

12. Anlage nach den Ansprüchen 1 bis 11, dadurch gekennzeichnet, daß die Rohrwendel (vgl. 44) mit beliebig verlaufenden Kühlrippen (48, 49, 50) ausgestattet ist.

13. Anlage nach den Ansprüchen 1 bis 12, dadurch gekennzeichnet, daß die Wirkung des Kühleinsatzes (46) in Abhängigkeit von der Temperatur des Biogases am Austritt aus dem Biogaskühler (41) gesteuert (vgl. 51) ist.

14. Anlage nach den Ansprüchen 1 bis 13, dadurch gekennzeichnet, daß in der Biogasführung (44) besondere Bremsstrecken (47) vorgesehen sind, die jedoch die stetige Steigung, also das Gefälle in der Biogaswendel nicht beeinträchtigen.

15. Anlage nach Anspruch 1 und/oder einem oder mehreren der folgenden Ansprüche, dadurch gekennzeichnet, daß in der Biogasleitung (45), bevorzugt hinter dem Biogaskühler (41) ein Schadstoffmengen- und/oder Qualitätsmeßgerät (52) vorgesehen ist.

16. Anlage nach Anspruch 1 und/oder einem oder mehreren der folgenden Ansprüche, dadurch gekennzeichnet, daß in der Biogasleitung (45) eine Art Gasuhr (53) vorgesehen ist, die eine Steuereinrichtung enthält, die einen gleichmäßigen Zufluß von Frischgülle aus einem dem Fermenter (1) vorgeschalteten Vorratsbehälter (55) in den Fermenter (1) regelt.

17. Anlage nach Anspruch 1 und/oder einem oder mehreren der folgenden Ansprüche, insbesondere nach Anspruch 15, dadurch gekennzeichnet, daß in der Biogasleitung (45), bevorzugt in Verbindung mit dem Qualitätsmeßgerät (vgl. 52) eine Einrichtung vorgesehen ist, die eine Pumpe (57, vgl. auch 58) steuert, die zwischen der an den Ablauf des Fermenters (1) angeschlossenen Entnahmestelle (29) für die ausgegorene Substanz und der Zufuhr (vgl. 9) von Frischgülle liegt.

18. Anlage nach Anspruch 1 und/oder einem oder mehreren der folgenden Ansprüche, dadurch gekennzeichnet, daß an die Biogasleitung (45) ein eine Feineinbringung von Luft zulassender Kleinkompressor (60) angeschlossen ist.

Fig.1

Fig.2

EP 0 501 376 A2

Fig.3

Fig.4

EP 0 501 376 A2

Fig.5

Fig.6

Fig.7

EP 0 501 376 A2